(19) Europäisches Patentamt
European Patent Office
Office européen des brevets

(11) Publication number: **0 058 567**
**B1**

# (12) EUROPEAN PATENT SPECIFICATION

(45) Date of publication of patent specification: **25.07.84**

(51) Int. Cl.³: **C 07 C 103/52, A 61 K 37/02**

(21) Application number: **82300794.3**

(22) Date of filing: **17.02.82**

## (54) Substituted acyl derivatives of octahydro-1H-isoindole-1-carboxylic acids and esters.

(30) Priority: **17.02.81 US 235381**
**07.12.81 US 327651**

(43) Date of publication of application:
**25.08.82 Bulletin 82/34**

(45) Publication of the grant of the patent:
**25.07.84 Bulletin 84/30**

(84) Designated Contracting States:
**AT BE CH DE FR GB IT LI LU NL SE**

(56) References cited:
**EP - A - 0 037 231**
**EP - A - 0 050 800**

(73) Proprietor: **WARNER-LAMBERT COMPANY**
**201 Tabor Road.**
**Morris Plains New Jersey 07950 (US)**

(72) Inventor: **Blankley, Clifton John**
**3724 Vorhies**
**Ann Arbor Michigan 48105 (US)**

(74) Representative: **Jones, Michael Raymond et al,**
**HASELTINE LAKE & CO. 28 Southampton**
**Buildings Chancery Lane**
**London WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 058 567 B1

## Description

This invention relates to substituted acyl derivatives of octahydro-1H-isoindole-1-carboxylic acids and esters.

The invention provides substituted acyl derivatives of octahydro-1H-isoindole-1-carboxylic acids and esters having the formula

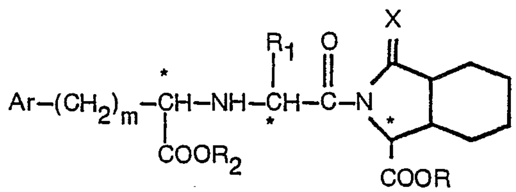

(I)

and pharmaceutically acceptable salts thereof, wherein R and $R_2$ are hydrogen or lower alkyl, $R_1$ is hydrogen, lower alkyl or benzyl, Ar is phenyl or phenyl substituted with one or two substituents selected from fluorine, chlorine, bromine, lower alkyl, lower alkoxy, hydroxy and amino, m is 0 to 3, and X is one oxygen atom. The terms lower alkyl and lower alkoxy include groups having straight or branched chains and containing 1 to 4 carbon atoms.

Preferred derivatives of the invention are acylated octahydro-1H-isoindole-1-carboxylic acids having the formula

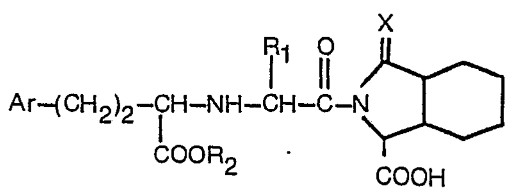

and the pharmaceutically acceptable salts thereof, wherein $R_1$ is hydrogen or lower alkyl containing 1 to 3 carbon atoms, $R_2$ is hydrogen or lower alkyl containing 1 to 3 carbon atoms, Ar is phenyl or phenyl substituted in the *para* position by fluorine, chlorine, bromine, methyl, hydroxy, methoxy or amino, and X is as defined above.

Further preferred derivatives of the invention are acylated octahydro-1H-isoindole-1-carboxylic acids having the formula

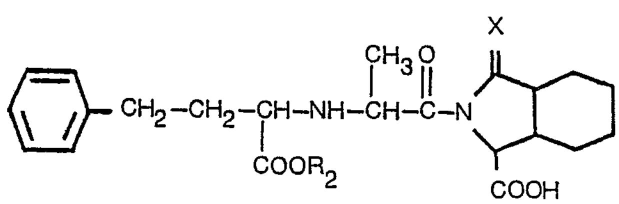

and the pharmaceutically acceptable salts thereof, wherein $R_2$ is hydrogen or lower alkyl of 1 to 3 carbon atoms, and X is as defined above, and specifically the compounds 2-[2-[(1-carboxy-3-phenylpropyl)amino]-1-oxopropyl]-octahydro-3-oxo-1H-isoindole-1-carboxylic acid, 2-[2-[(1-carbethoxy-3-phenylpropyl)amino]-1-oxopropyl]-octahydro-3-oxo-1H-isoindole-1-carboxylic acid, and the pharmaceutically acceptable salts thereof.

The compounds of the invention have asymmetric carbon atoms. These carbon atoms are indicated by an asterisk in formula I. Additional asymmetric carbon atoms may be present in the lower alkyl groups. The compounds accordingly exist as optical isomers and diastereomers or as racemates and mixtures thereof. All of these are within the scope of the invention. The *S* configuration at the centres marked with an asterisk in formula I is preferred.

The octahydro-1H-isoindole-3-oxo-1-carboxylic acid nuclei of the compounds of this invention may potentially exist in several isomeric forms. The stereochemistry at the fused ring junction is believed to be *cis*. The carboxylic acid group may be either *cis* or *trans* to the fused cyclohexane ring.

The compounds of the invention may exist in anhydrous form as well as in solvated, including hydrated, forms. In general, the hydrated forms and the solvated forms with pharmaceutically acceptable solvents are equivalent to the anhydrous or unsolvated form for the purposes of the invention.

The compounds of formula I can be prepared from octahydro-1H-isoindole-3-oxo-1-carboxylic acids of formula III:

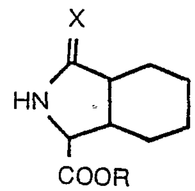

(III)

Thus, for example, the compounds of formula I may be prepared from octahydro-1H-isoindole-3-oxo-1-carboxylic acids III by first protecting the carboxylic acid group, preferably as an ester, e.g. with a lower alkyl, benzyl or trimethylsilyl group. The protectec carboxylic acid compound is coupled to an N-protected amino acid, e.g. glycine or L-alanine, protected on nitrogen, e.g. with *t*-butyloxycarbonyl or benzyloxycarbonyl. The coupling is carried out by any of a variety of standard peptide coupling techniques as disclosed, for example, in "The Peptides. Analysis, Synthesis, Biology, Vol. 1, Major Methods of Peptide Bond Formation, Part A," ed. E. Gross, J. Meierhofer, Academic Press N.Y. (1979). An especially useful method involves the use of a dehydrating agent, such as dicyclohexyl-carbodiimide alone or in the presence of reagents forming reactive esters, e.g. 1-hydroxybenztriazole, in suitable aprotic solvents such as dimethylformamide, acetonitrile, tetrahydrofuran or chlorinated hydrocarbons. This gives the intermediate N-protected-(2-aminoacyl) octahydro-1H-isoindole-1-carboxylic acid esters. These may then be either partially or totally deblocked depending on the protecting groups chosen, using anhydrous acids, e.g., hydrochloric acid in acetic acid or trifluoroacetic acid in dichloromethane or hydrogen gas and a catalyst to give the intermediate dipeptide either in free form or protected as an ester.

The compounds of the invention of formula I may then be prepared by reacting the intermediate dipeptide or its ester derivative with $\alpha$-keto-4-substituted phenylbutyric acid or its lower alkyl ester derivatives under dehydrating and reducing conditions. Preferred dehydrating agents include molecular sieves in aprotic solvents and preferred reducing agents include sodium cyanoborohydride or hydrogen gas with a catalyst.

Alternatively, the dipeptide or its ester derivative may be reacted with an $\alpha$-halo-4-substituted phenylbutyric acid or its ester in the presence of a suitable basic reagent, such as triethylamine or alkali carbonates or bicarbonates, in a solvent, to give the compounds of the invention of formula I. Ester protected products may be hydrolyzed under basic or acidic reaction conditions to free acid derivatives, or, in the case of benzyl esters, catalytic hydrogenolysis may be preferred.

Alternately, compounds of the invention of formula I may be prepared in a different manner. This consists of applying either of the two methods described above for the attachment of the 2-(4-phenylbuyric acid) moiety to the protected dipeptide, first to glycine or L-alanine, protected as an ester, to give N-[2-(4-phenylbutyric acid)]-substituted glycine or L-alanine derivative.

After selective deblocking of the acid moiety on the glycine or alanine portion of the product, the resulting monoacid may be coupled, either directly or subsequent to suitable blocking of the amino group, via standard peptide coupling procedures to the octahydro-1H-isoindole-1-carboxylic acids III protected as an ester, i.e., $R_3$. Selective or complete removal of the ester groups and any amine protecting groups yield the compounds of formula I.

The products are obtained typically as a mixture of diastereomers which can be separated by standard methods of fractional crystallization or chromatography.

The compounds of this invention form basic salts with various inorganic and organic bases which are also within the scope of the invention. Such salts include ammonium salts, alkali metal salts like sodium and potassium salts, alkaline earth metal salts like the calcium and magnesium salts, salts with organic bases, e.g., dicyclohexylamine or benzathine, salts with basic amino acids like arginine, lysine and the like. The pharmaceutically acceptable salts are preferred, although other salts such as the dicyclohexylamine salt are also useful, e.g., in isolating, purifying or characterizing the product.

The salts are formed in conventional manner by reacting the free acid form of the product with one or more equivalents of the appropriate base providing the desired cation in a solvent of medium in which the salt is insoluble, or in water and removing the water by freeze drying.

In the compounds of formula I the pharmaceutically acceptable acid addition salts may be prepared by conventional reactions with equivalent amounts of organic or inorganic acids. As exemplary, but not limiting, of pharmaceutically acceptable acid salts are the salts of hydrochloric, sulfuric, acetic, fumaric, malic, maleic and citric acids.

The action of the enzyme resin on angiotensinogen, a pseudoglobulin in blood plasma, produces the decapeptide antiotensin I. Antiotensin I is converted by antiotensin converting enzyme (ACE) to the octapeptide angiotensin II. The latter is an active pressor substance which has been implicated as the causative agent in various forms of hypertension in various mammalian species. e.g., rats and dogs. The compounds of this invention intervene in the renin→angiotensin I→angiotensin II sequence by inhibiting angiotensin I converting enzyme and reducing or eliminating the formation of the pressor substance angiotensin II, and therefore are useful in reducing or relieving hypertension. Thus by the administration of a composition containing one or a combination of compounds of formula I or a pharmaceutically acceptable salt thereof, hypertension in the species of mammal suffering therefrom is alleviated. A single dose, or preferably two to four divided daily doses, provided on a basis of about 0.1 to 100 mg per kilogram per day, preferably about 1 to 50 mg per kilogram per day is appropriate to reduce blood pressure. The substance is preferably administered orally, but parenteral routes such as subcutaneously, intramuscularly, intravenously or intraperitonealy can also be employed.

The following Table shows the *in vitro* activity of compounds of formula IV in an assay angiotensin converting enzyme inhibitory activity which is a modification of a test reported by D. Cushman and H. Cheung, Biochemical Pharmacology, *20*, 1637—1648 (1971).

*In vitro ACE Assay:* Angiotensin converting enzyme (ACE) inhibitory activity is determined by assaying guinea pig serum ACE in the presence and absence of the test compound. ACE from guinea pig serum and the test compounds are preincubated for 10 minutes before the addition of the labelled substrate $^3$H-hippuryl-glycyl-glycine. After a 60 minute incubation at 37°C the reaction is stopped by the addition of 0.1 N HCl. ACE cleaves the hippuryl-glycyl bond to form the dipeptide glycyl-glycine and $^3$H-hippuric acid. The $^3$H-hippuric acid is then extracted with ethyl acetate and the ACE inhibition of a given sample calculated on the basis of the $^3$H-hippuric acid generated.

Table

Activity of Compounds of Formula IV

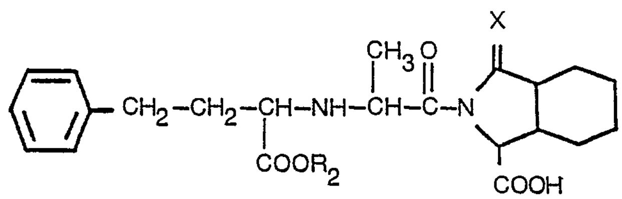

| $R_2$ | X | $IC_{50}$ |
|---|---|---|
| $CH_2CH_3$ | 0 | 0.34 |

The $IC_{50}$ is the molar concentration of compound which inhibits 50% of the conversion of angiotensin *I* to angiotension *II*.

The compounds of the invention can be utilized to reduce blood pressure in the form of tablets, capsules or elixirs for oral administration or in sterile solutions or suspensions for parenteral administration. About 10 to 500 mg. of a compound or mixture of compounds of formula I or a pharmaceutically acceptable salt is compounded with a pharmaceutically acceptable vehicle or carrier which may contain excipients, binders, preservatives, stabilizers, flavors, etc., in accord with accepted pharmaceutical practice. The amount of active substance in these compositions or preparations is such that a suitable dosage in the range indicated is obtained.

Illustrative of the inert ingredients which may be incorporated in tablets, capsules and the like are the following: A binder such as gum tragacanth, acacia, corn starch or gelatin; an excipient such as dicalcium phosphate; a disintegrating agent such as corn starch, potato starch, alginic acid and the like; a lubricant such as magnesium stearate; a sweetening agent such as sucrose, lactose or saccharin; a flavoring agent such as peppermint, oil of wintergreen or cherry. When the dosage unit form is a capsule, it may contain in addition to materials of the above type a liquid carrier such as a fatty oil. Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a vehicle such as water for injection, a naturally occurring vegetable oil like sesame oil, coconut oil, peanut oil, cottonseed oil, etc., or a synthetic fatty vehicle like ethyl oleate or the like. Buffers, preservatives, antioxidants and the like can be incorporated as required.

The invention is illustrated by the following examples.

Example 1 (comparative)

2-[2-[(1-Carbethoxy-3-phenylpropyl)amino]-1-oxopropyl]-octahydro-1H-isoindole-1-carboxylic acid, hydrochloride.

A mixture of 6.5 g of t-butyl octahydro-1H-isoindole-1-carboxylate hydrochloride, 7.0 g of (*S, S*) ethyl $\alpha$-[(1-carboxyethyl)amino]benzenebutanoate (prepared by neutralization of the hydrochloride

salt), 3.4 g of hydroxybenztriazole and 2.5 g of triethylamine in 125 ml of tetrahydrofuran is stirred and cooled to 0°C while a solution of 5.2 g of dicyclohexylcarbodiimide in 15 ml of tetrahydrofuran is added dropwise. The mixture is stirred at 0°C for one hour, and then allowed to warm to room temperature overnight. The mixture is filtered and the filtrate is concentrated at reduced pressure. The residue is taken up in ethyl acetate and the mixture is filtered again, washed successively with saturated sodium bicarbonate solution and saturated sodium chloride solution, dried and concentrated at reduced pressure to give product as an oily mixture of two isomers. The desired, more active isomer (presumed to have the *S* configuration at centers designated with an asterisk in formula I) is separated after chromatography over silica gel, eluting with a 99:1 chloroform/methanol mixture and collecting the product isomer first eluted. This is an oil with $[\alpha]_D^{23} = -45.0°$ (c = 1.08, ethanol).

This preferred diester is converted to the corresponding monoethyl ester by dissolving it in methylene chloride, filtering to clarify the solution, cooling to 0°C and bubbling hydrochloric acid gas through the solution for 15 minutes. After standing overnight at 0°C, the solution is concentrated to remove solvent. The residue is crystallized by treating an ethanol solution of it with ether. The desired product, (*S, S, S*)-2-[2-[(1-carbethoxy-3-phenylpropyl)amino]-1-oxopropyl]octahydro-1H-isoindole-1-carboxylic acid, hydrochloride has mp 198—216° (dec.); $[\alpha]_D^{23} = -33.6°$ (c = 1.07, ethanol), and $R_f = 0.09$ (silica gel; 95:1 chloroform/methanol, developed twice).

The intermediate (*S, S*) ethyl $\alpha$-[(1-carboxyethyl)-amino]benzenebutanoate hydrochloride used in this preparation may be prepared in the following manner. A solution of 2.0 g of t-butyl L-alanine and 3.78 g of ethyl 2-bromo-4-phenylbutanoate in 25 ml of dimethylformamide is treated with 1.8 ml of triethylamine and the solution is heated at 70°C for 18 hours. The solvent is removed at reduced pressure and the residue is mixed with water and extracted with ethyl ether. The organic layer is washed with water and dried over magnesium sulfate. Concentration of the solvent at reduced pressure gives the oily t-butyl ester of the intermediate which is found to be sufficiently pure by gas liquid chromatography for further use.

A solution of 143.7 g of this t-butyl ester in 630 ml of trifluoroacetic acid is stirred at room temperature for one hour. The solvent is removed at reduced pressure and the residue is dissolved in ethyl ether and again evaporated. This operation is repeated. Then the ether solution is treated dropwise with a solution of hydrogen chloride gas in ethyl ether until precipitation ceases. The solid is collected by filtration and is a mixture of diastereoisomers, mp 153—165°C, $[\alpha]_D^{23} = +3.6°$ (c = 1, methanol).

In order to separate the preferred *S, S* isomer, a suspension of 10.0 g of the mixture in 200 ml of methylene chloride is stirred at room temperature for five minutes and filtered; the solid is washed with additional methylene chloride and finally ether. The solid material, mp 202—208°C (dec.), $[\alpha]_D^{23} = -29.3°$ (c = 1, methanol) is the less preferred diastereoisomer having the *R, S* configuration (*S* referring to the portion derived from L-alanine). The preferred *S, S* diastereoisomer can be recovered from the filtrate after concentration and trituration of the residue with ether. It has mp 137—139°C, $[\alpha]_D^{23} = +31.3°$ (c = 1, methanol).

The butyl octahydro-1H-isoindole-1-carboxylate hydrochloride is prepared as follows. Following a procedure given in Gazz. Chim. Ital, *106*, 65(1976), 56 g of (2-methylphenyl)acetyl chloride is converted to 2-bromo(2-bromomethylphenyl)acetyl chloride. This is added dropwise to a solution of 78.6 g of t-butyl alcohol in 120 ml of ether and cooled at 0°C. Finally 26.4 g of pyridine is added. After a few minutes, the precipitated solids are filtered and washed with ether. The filtrate is washed successively with water and dilute sodium bicarbonate solution dried and concentrated at reduced pressure. t-Butyl 2-bromo-(2-bromomethylphenyl) acetate is obtained as an oil of sufficient purity for further use by chromatography over silica gel, eluting with a 4:1 hexane/ethyl acetate mixture.

A mixture of 11.5 g of this ester and 15 g of powdered anhydrous sodium bicarbonate in 100 ml of acetonitrile is cooled to 0°C under nitrogen and treated dropwise with 3.7 g of benzylamine. The mixture is allowed to stand at room temperature for 64 hours, then filtered. Concentration gives crude t-butyl 2,3-dihydro-2-(phenylmethyl)-1H-isoindole-1-carboxylate which is purified by conversion to the hydrochloride salt with hydrogen chloride gas in ether. The salt has mp 154—157°C after recrystallization from ethyl acetate.

The N-benzyl group is removed from this salt by hydrogenolysis in methanol solution over 20% Pd/C catalyst. The product, t-butyl 2,3-dihydro-1H-isoindole-1-carboxylate hydrochloride, is recrystallized from ethanol/ether and has mp 148—156°C (decomposition).

A solution of 13.8 g of this salt in 300 ml of a 7:1 t-butyl alcohol/tetrahydrofuran mixture is treated with 2 g of a 10% Rh/C catalyst and hydrogenated at 40°C and 50 psi initial pressure. Additional time and catalyst may be required if hydrogen uptake is not complete. The desired t-butyl octahydro-1H-isoindole-1-carboxylate hydrochloride is obtained after filtration of the catalyst and concentration of the filtrate. It may be purified by recrystallization from ethanol/ether to give material with mp 173—176°C.

Example 2 (comparative)

2-[2-[(1-Carboxy-3-phenylpropyl)amino]-1-oxopropyl]-octahydro-1H -isoindole-1-carboxylic acid

A solution of 0.7 g of 2-(2-([1-carbethoxy-3-phenylpropyl]amino]-1-oxopropyl]octahydro-1H-isoindole-1-carboxylic acid hydrochloride in 15 ml of absolute ethanol is treated with 0.5 g of potassium

hydroxide pellets. The mixture is stirred at room temperature overnight, then filtered and the solvent is evaporated at reduced pressure. The residue is taken up in 50 ml of water and acidified to pH 3 with 4N hydrochloric acid. The product precipitates out and is recrystallized from absolute ethanol; mp 193—197°C $[\alpha]_D^{23} = -39.9°$.

Example 3

2-[2-[(1-carbethoxy-3-phenylpropyl)amino]-1-oxopropyl]-octahydro-3-oxo-1H-isoindole-1-carboxylic acid, hydrochloride

Following the procedure of Example 1, but substituting t-butyl octahydro-3-oxo-1H-isoindole-1-carboxylate for 5-butyl octahydro-1H-isoindole-1-carboxylate gives the desired product as a mixture of isomers. This is further purified by dissolving it in water, filtering insolubles, and freeze-drying the filtrate. This gives an amorphous solid, mp 50—70° (shrinks), 160° (decompose) with $[\alpha]_D^{23} = +9.5°$ (C = 1.01, ethanol), $R_f = 0.05$, 0.1 (silica gel; 95:1 chloroform/methanol, developed twice).

The intermediate t-butyl octahydro-3-oxo-1H-isoindole-1-carboxylate is prepared from octahydro-3-oxo-1H-isoindole-carboxylic acid as follows. A mixture of 10 g of this acid and 0.4 ml of 70% perchloric acid in 150 ml of t-butyl acetate is allowed to stand for 2.5 days. An additional 50 ml of t-butyl acetate is added and the reaction is allowed to continue for 15 days. The solution is filtered and poured onto 14 g of 50% sodium hydroxide solution and ice. The product is extracted into ether. Drying and concentration gives a residue which when triturated with hexane gives material, mp 143—149°C, suitable for further use. The octahydro-3-oxo-1H-isoindole-1-carboxylic acid from which the intermediate is prepared may in turn be prepared as follows. 2,3-Dihydro-3-oxo-1H-isoindole-1-carboxylic acid (J. Prakt. Chem., *146*, 307(1936)), 20 g is heated at reflux for 2 hours with 250 ml of absolute ethanol and 5.6 g of concentrated sulfuric acid. On cooling, the solution deposits crystalline ethyl 2,3-dihydro-3-oxo-1H-isoindole-3-carboxylate. Recrystallization of this from ethanol gives pure material, mp 178—183°C. This ester, 15 g, is dissolved in 110 ml of tetrahydrofuran and 110 ml of absolute ethanol, treated with 1.0 g of 10% Rh/C catalyst and hydrogenated at an initial pressure of 50 psi and 36°C. After the required amount of hydrogen has been absorbed, the mixture is filtered and the filtrate is concentrated to give the crude product. Pure ethyl octahydro-3-oxo-1H-isoindole-1-carboxylate has mp 149—157°C after recrystallization from ethanol. This ester, 5 g, is hydrolyzed by treating it with a mixture of 48 ml 1N sodium hydroxide and 10 ml of ethanol overnight at room temperature. Partial concentration to remove ethanol and acidification precipitates the crude acid. Octahydro-3-oxo-1H-isoindole-1-carboxylic acid has mp 205—213°C (decomposition) after recrystallization from water.

**Claims for the Contracting States: BE CH DE FR GB IT LI LU NL SE**

1. A substituted acyl derivative of an octahydro-1H-isoindole-1-carboxylic acid or ester having the formula

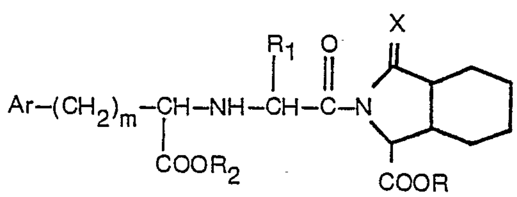

wherein R is hydrogen or lower alkyl, $R_1$ is hydrogen, lower alkyl or benzyl, $R_2$ is hydrogen or lower alkyl, Ar is phenyl or phenyl substituted by one or two substituents selected from fluorine, chlorine, bromine, lower alkyl, lower alkoxy, hydroxy and amino, m is 0 to 3, and X is one oxygen atom, and wherein lower alkyl and lower alkoxy include straight or branched groups containing 1 to 4 carbon atoms, or a pharmaceutically acceptable basic salt thereof.

2. A derivative according to claim 1, having the formula

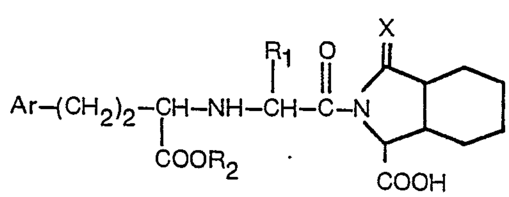

wherein $R_1$ is hydrogen or lower alkyl containing 1 to 3 carbon atoms, $R_2$ is hydrogen or lower alkyl containing 1 to 3 carbon atoms, Ar is phenyl or phenyl substituted in the *para* position by fluorine, chlorine, bromine, methyl, hydroxy, methoxy or amino, and X is as defined in claim 1, or a pharmaceutically acceptable salt thereof.

3. A derivative according to claim 2, having the formula

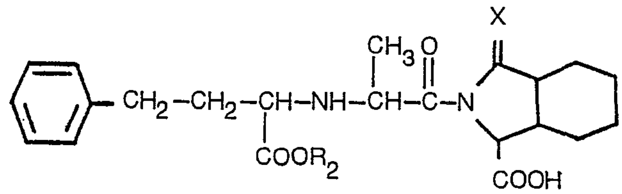

wherein $R_2$ is hydrogen or lower alkyl containing 1 to 3 carbon atoms, and X is as defined in claim 1, or a pharmaceutically acceptable salt thereof.

4. 2-[2-[(1-Carboxy-3-phenylpropyl)amino]-1-oxopropyl]-octahydro-3-oxo-1H-isoindole-1-carboxylic acid or a pharmaceutically acceptable salt thereof.

5. 2-[2-[(1 Carbethoxy-3-phenylpropyl)amino]-1-oxopropyl]-octahydro-3-oxo-1H-isoindole-1-carboxylic acid or a pharmaceutically acceptable salt thereof.

6. A process for the production of a substituted acyl derivative of octahydro-1H-isoindole-1-carboxylic acid according to claim 1, which comprises peptide coupling of a protected octahydro-1H-isoindole-1-carboxylate of the formula

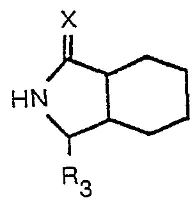

wherein X is as defined in claim 1 and $R_3$ is a protected carboxylic acid group, with an N-substituted amino acid of the formula

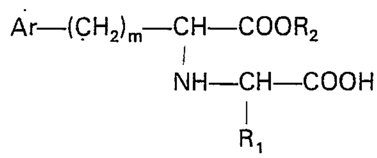

where $R_1$, Ar and m are as defined in claim 1 and $R_2$ is lower alkyl, and removing the protective group.

7. A pharmaceutical composition comprising a substituted acyl derivative of octahydro-1H-isoindole-1-carboxylic acid according to any of claims 1 to 5, or a pharmaceutically acceptable salt thereof according to any of claims 1 to 5, and a pharmaceutically acceptable carrier.

**Claims for the Contracting State: AT**

1. A process for producing a substituted acyl derivative of isoindole-1-carboxylic acid or ester having the formula

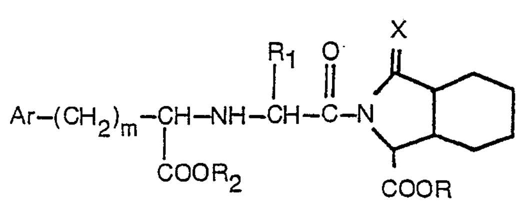

wherein R is hydrogen or lower alkyl, $R_1$ is hydrogen, lower alkyl or benzyl, $R_2$ is hydrogen or lower alkyl, Ar is phenyl or phenyl substituted by one or two substituents selected from fluorine, chlorine, bromine, lower alkyl, lower alkoxy, hydroxy and amino, m is 0 to 3, and X is one oxygen atom, and wherein lower alkyl and lower alkoxy include straight or branched groups containing 1 to 4 carbon atoms, which process comprises (1) peptide coupling of a protected octahydro-1H-isoindole-1-carboxylate having the formula

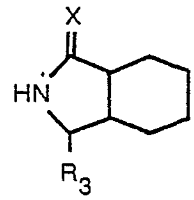

where $R_3$ is a protected carboxylic acid group, with an N-substituted amino acid having the formula

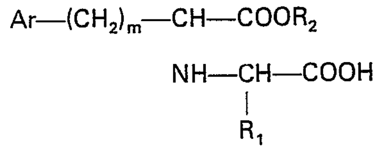

wherein $R_1$, $R_2$, Ar and m are as previously defined, and (2) removing the protective group.

2. A process for producing a substituted acyl derivative of an octahydro-1H-isoindole-1-carboxylic acid or ester having the formula

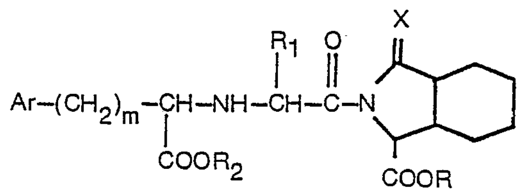

wherein Ar, R, $R_1$, $R_2$, m and X are as defined in claim 1, which process comprises reacting a compound having the formula

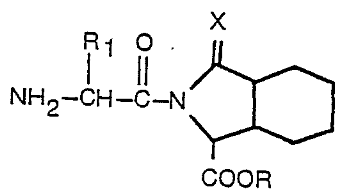

wherein R, $R_1$ and X are as defined in claim 1, with a compound having the formula

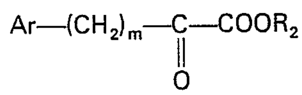

or the formula

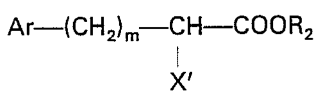

where Ar, $R_2$ and m are as defined in claim 1 and X′ is halogen.

3. A process for producing a pharmaceutically acceptable salt of a compound produced by a process according to claim 1 or 2, which process comprises reacting the compound with a base or acid capable of forming a pharmaceutically acceptable salt.

4. A process according to any of claims 1 to 3, being a process for producing a derivative having the formula

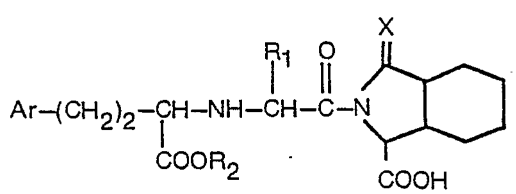

wherein $R_1$ is hydrogen or lower alkyl containing 1 to 3 carbon atoms, $R_2$ is hydrogen or lower alkyl containing 1 to 3 carbon atoms, Ar is phenyl or phenyl substituted in the *para* position by fluorine, chlorine, bromine, methyl, hydroxy, methoxy or amino; and X is as defined in claim 1, or a pharmaceutically acceptable salt thereof.

5. A process according to any of claims 1 to 3, being a process for producing a derivative having the formula

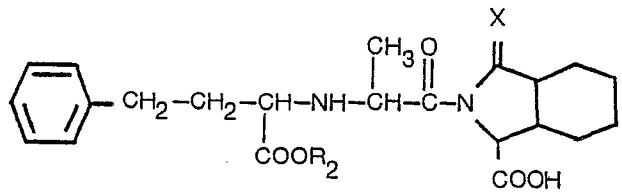

wherein $R_2$ is hydrogen or lower alkyl containing 1 to 3 carbon atoms, and X is as defined in claim 1, or a pharmaceutically acceptable salt thereof.

6. A process according to any of claims 1 to 3, being a process for producing the compound 2-[2-[(1-carboxy-3-phenylpropyl)amino]-1-oxopropyl]-octahydro-3-oxo-1H-isoindole-1-carboxylic acid or a pharmaceutically acceptable salt thereof.

7. A process according to any of claims 1 to 3, being a process for producing the compound 2-[2-[(1-carbethoxy-3-phenylpropyl)amino]-1-oxopropyl]-octahydro-3-oxo-1H-isoindole-1-carboxylic acid or a pharmaceutically acceptable salt thereof.

**Revendications pour les Etats Contractants: BE CH DE FR GB IT LI LU NL SE**

1. Un dérivé acylique substitué d'un acide octahydro-1H-isoindole-1-carboxylique ou ester de cet acide présentant la formule

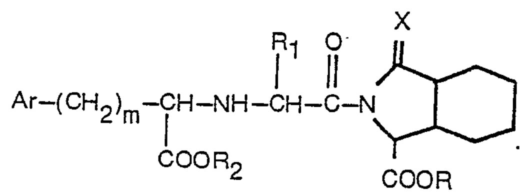

dans laquelle R est de l'hydrogène ou un alkyle inférieur; $R_1$ est de l'hydrogène, un alkyle inférieur ou benzyle; $R_2$ est de l'hydrogène, ou un alkyl inférieur; Ar est un phényle ou un phényle substitué par un ou deux substituants choisis parmi fluor, chlore, brome, alkyle inférieur, alkoxy inférieur, hydroxy et amino; m est égal à 0 à 3; et X est un atome d'oxygène, et où l'alkyle inférieur ou l'alkoxy inférieur est formé de groupes linéaires ou ramifiés contenant de 1 à 4 atomes de carbone, ou un de ses sels d'une base acceptable pharmaceutiquement en dérivant.

2. Un dérivé selon la revendication 1, présentant la formule

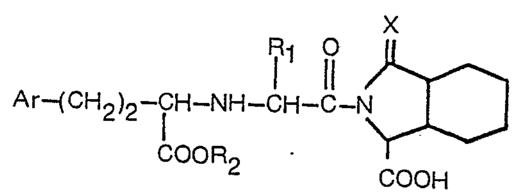

dans laquelle: $R_1$ est de l'hydrogène ou un alkyle inférieur contenant 1 à 3 atomes de carbone; $R_2$ est de l'hydrogène ou un radical alkyle inférieur contenant 1 à 3 atomes de carbone; Ar est un phényle ou un phényle substitué en position para par fluor, chlore, brome, méthyle, hydroxy, méthoxy ou amino, et X est tel que défini dans la revendication 1, ou un de ses sels acceptables du point de vue pharmaceutique en dérivant.

3. Un dérivé selon la revendication 2, présentant la formule

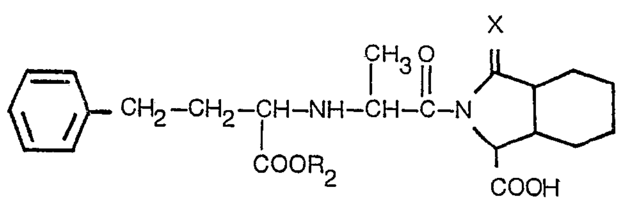

dans laquelle: $R_2$ est l'hydrogène ou un alkyle inférieur contenant 1 à 3 atomes de carbone; X est tel que défini dans la revendication 1, ou un de ses sels acceptables du point de vue pharmaceutique.

4. L'acide 2-{2-[(1-carboxy-3-phénylpropyl)amino]-1-oxopropyl}-octahydro-3-oxo-1H-isoindole-1-carboxylique ou un de ses sels acceptables du point de vue pharmaceutique.

5. L'acide 2-{2-[(-carbéthoxy-3-phénylpropyl)amino]-1-oxopropyl}-octahydro-3-oxo-1H-isoindole-1-carboxylique ou un de ses sels acceptables du point de vue pharmaceutique.

6. Un procédé de production d'un dérivé acylique substitué de l'acide octahydro-1H-isoindole-1-carboxylique selon la revendication 1, qui consiste à coupler un peptide d'un octahydro-1H-isoindole-1-carboxylate protégé de formule

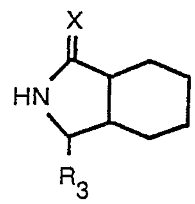

dans laquelle: X est tel que défini dans la revendication 1; et $R_3$ est un groupe acide carboxylique protégé avec un acide amino N-substitué de formule

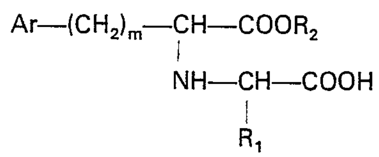

dans laquelle: $R_1$, Ar et m sont tels que définis ci-dessus; et $R_2$ est un alkyle inférieur, et élimination du groupe protecteur.

7. Une composition pharmaceutique comprenant un dérivé acide subtitué de l'acide octahydro-2H-isoindole-1-carboxylique selon l'une quelconque des revendications 1 à 5, ou un de ses sels acceptables du point de vue pharmaceutique, selon une quelconque des revendications 1 à 5, et d'un support acceptable du point de vue pharmaceutique.

**Revendications pour l'Etat Contractant: AT**

1. Un procédé de production d'un dérivé acylique substitué de l'acide isoindole-1-carboxylique ou d'un de ses esters présentant la formule

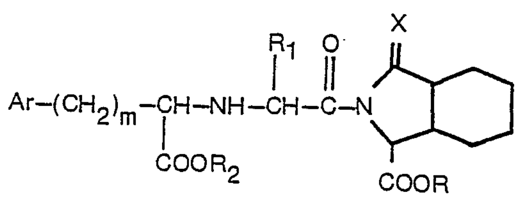

dans laquelle: R est l'hydrogène ou un alkyle inférieur; $R_1$ est l'hydrogène, un alkyle inférieur ou benzyle; $R_2$ est l'hydrogène ou un radical alkyle inférieur; Ar est un phényle, ou un phényle substitué par un ou deux substituants choisis parmi fluor, chlore, brome, alkyle inférieur, alkoxy inférieur, hydroxy et amino; m est 0 à 3; X est un atome d'oxygène; et dans lequel l'alkyl inférieur et l'alkoxy inférieur consistent en des groupes linéaires ou ramifiés contenant 1 à 4 atomes de carbone, lequel procédé consiste: (1) à coupler un peptide d'un octahydro-1H-isoindole-1-carboxylate protégé présentant la formule

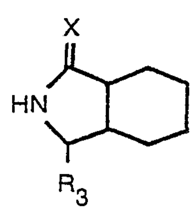

dans laquelle: $R_3$ est un groupe acide carboxylique protégé, avec un amino acide N-substitué présentant la formule:

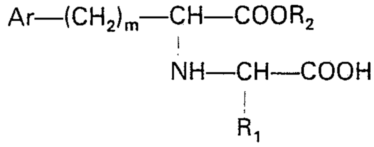

dans laquelle: $R_1$, $R_2$, Ar et m sont tels que définis ci-dessus; et (2) éliminer le groupe protecteur.

2. Un procédé de production d'un dérivé acylique d'un acide octahydro-1H-isoindole-1-carboxylique ou un de ses esters présentant la formule

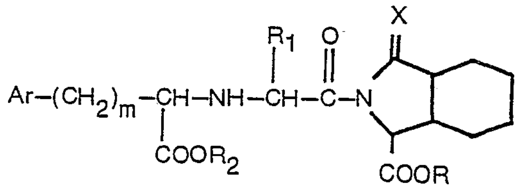

dans laquelle: Ar, R, $R_1$, $R_2$, m et X sont tels que définis dans la revendication 1, lequel procédé consiste à faire réagir un composé présentant la formule

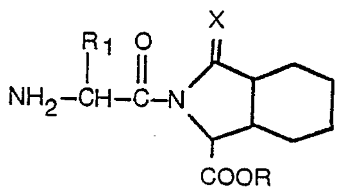

dans laquelle: R, $R_1$ et X sont tels que définis dans la revendication 1, avec un composé présentant la formule

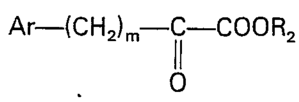 ou 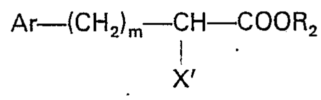

dans laquelle: Ar, $R_2$ et m sont tels que définis dans la revendication 1; et X' est un halogène.

3. Un procédé de production d'un sel acceptable du point de vue pharmaceutique d'un composé produit par un procédé selon la revendication 1 ou 2, ce procédé consistant à faire réagir le composé avec une base ou un acide susceptible de former un sel acceptable du point de vue pharmaceutique.

4. Un procédé selon l'une quelconque des revendications 1 à 3, consistant en un procédé de production d'un dérivé et présentant la formule

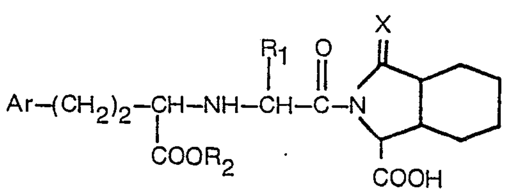

dans laquelle: $R_1$ est l'hydrogène ou un alkyle inférieur contenant 1 à 3 atomes de carbone; $R_2$ est l'hydrogène ou un alkyle inférieur contenant 1 à 3 atomes de carbone; Ar est un phényle, ou un phényle substitué en position para par fluor, chlore, brome, méthyle, méthoxy, hydroxy ou amino; et X est tel que défini dans la revendication 1; ou un de ses sels acceptables du point de vue pharmaceutique.

5. Un procédé selon l'une quelconque des revendications 1 à 3, consistant en un procédé de production d'un dérivé présentant la formule

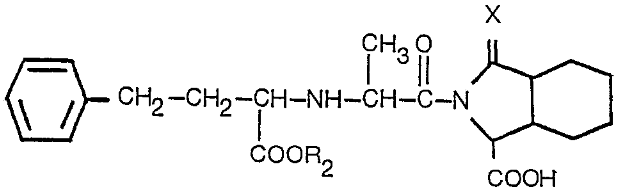

dans laquelle: $R_2$ est l'hydrogène ou un alkyle inférieur contenant 1 à 3 atomes de carbone; et X est tel que défini dans la revendication 1; ou un de ses sels acceptables du point du vue pharmaceutique.

6. Un procédé selon l'une quelconque des revendications 1 à 3, consistant en un procédé de production du composé consistant en l'acide 2-{2-[(1-carboxy-3-phénylpropyl)amino]-1-oxopropyl}-octahydro-3-oxo-1H-isoindole-1-carboxylique ou un de ses sels acceptables du point de vue pharmaceutique.

7. Un procédé selon l'une quelconque des revendications 1 à 3, consistant en un procédé de production du composé consistant en l'acide 2-{2-[(1-carbéthoxy-3-phénylpropyl)amino]-1-oxopropyl}-octahydro-3-oxo-1H-isoindole-1-carboxylique ou un de ses sels acceptables du point de vue pharmaceutique.

**Patentansprüche für die Vertragsstaaten: BE CH DE FR GB IT LI LU NL SE**

1. Substituiertes Acylderivat von Octahydro-1H-isoindol-1-carbonsäure oder -ester mit der Formel

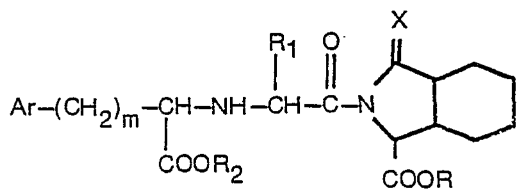

worin R Wasserstoff oder Niedrigalkyl bedeutet, $R_1$ Wasserstoff, Niedrigalkyl oder Benzyl bedeutet, $R_2$ Wasserstoff oder Niedrigalkyl bedeutet, Ar für Phenyl oder durch einen oder zwei Substituenten, ausgewählt aus Fluor, Chlor, Brom, Niedrigalkyl, Niedrigalkoxy, Hydroxy und Amino, substituiertes Phenyl steht, m 0 bis 3 bedeutet und X ein Sauerstoffatom bedeutet, und worin Niedrigalkyl und Niedrigalkoxy gerade oder verzweigte Gruppen mit 1 bis 4 Kohlenstoffatomen umfassen, oder ein pharmazeutisch akzeptables basisches Salz desselben.

2. Derivat nach Anspruch 1 mit der Formel

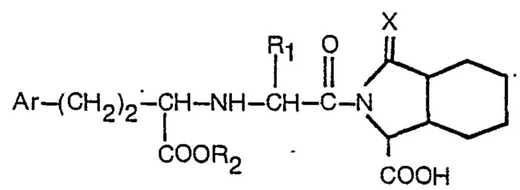

worin $R_1$ Wasserstoff oder Niedrigalkyl mit 1 bis 3 Kohlenstoffatomen bedeutet, $R_2$ Wasserstoff oder Niedrigalkyl mit 1 bis 3 Kohlenstoffatomen bedeutet, Ar Phenyl oder an der para-Position durch Fluor, Chlor, Brom, Methyl, Hydroxy, Methoxy oder Amino substituiertes Phenyl bedeutet und X der in Anspruch 1 angeführten Definition entspricht, oder ein pharmazeutisch akzeptables Salz desselben.

3. Derivat nach Anspruch 2 mit der Formel

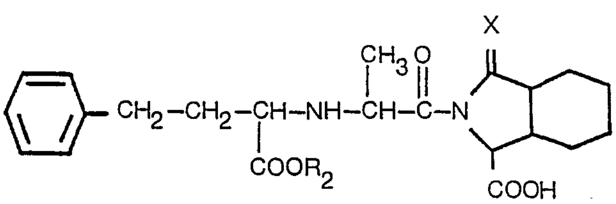

worin $R_2$ Wasserstoff oder Niedrigalkyl mit 1 bis 3 Kohlenstoffatomen bedeutet und X der in Anspruch 1 angegebenen Definition entspricht, oder ein pharmazeutisch akzeptables Salz desselben.

4. 2-[2-[(1-Carboxy-3-phenylpropyl)-amino]-1-oxopropyl]-octahydro-3-oxo-1H-isoindol-1-carbonsäure oder ein pharmazeutisch akzeptables Salz derselben.

5. 2-[2-[(1-Carbäthoxy-3-phenylpropyl)-amino]-1-oxopropyl]-octahydro-3-oxo-1H-isoindol-1-carbonsäure oder ein pharmazeutisch akzeptables Salz derselben.

6. Verfahren zur Herstellung eines substituierten Acylderivates der Octahydro-1H-isoindol-1-carbonsäure nach Anspruch 1, dadurch gekennzeichnet, daß ein geschütztes Octahydro-1H-isoindol-1-carboxylat der Formel

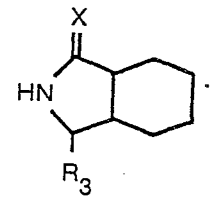

worin X der in Anspruch 1 angegebenen Definition entspricht und $R_3$ eine geschützte Carbonsäuregruppe bedeutet, mit einer N-substituierten Aminosäure der Formel

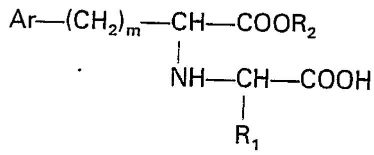

worin $R_1$, Ar und m der in Anspruch 1 angegebenen Definition entsprechen und $R_2$ Niedrigalkyl bedeutet, einer Peptidkupplung unterzogen wird und die Schutzgruppe entfernt wird.

7. Pharmazeutische Zusammensetzung umfassend ein substituiertes Acylderivat der Octahydro-1H-isoindol-1-carbonsäure nach irgendeinem der Ansprüche 1 bis 5 oder ein pharmazeutisch akzeptables Salz desselben nach irgendeinem der Ansprüche 1 bis 5 und einen pharmazeutisch akzeptablen Träger.

**Patentansprüche für den Vertragsstaat: AT**

1. Verfahren zur Herstellung eines substituierten Acylderivats von Isoindol-1-carbonsäure oder -ester mit der Formel

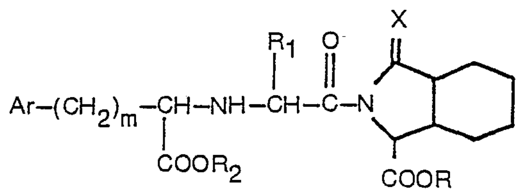

worin R Wasserstoff oder Niedrigalkyl bedeutet, $R_1$ Wasserstoff, Niedrigalkyl oder Benzyl bedeutet, $R_2$ Wasserstoff oder Niedrigalkyl bedeutet, Ar Phenyl oder durch einen oder zwei Substituenten, ausgewählt aus Fluor, Chlor, Brom Niedrigalkyl, Niedrigalkoxy, Hydroxy und Amino, substituiertes Phenyl bedeutet, m 0 bis 3 bedeutet und X ein Sauerstoffatom bedeutet, und worin Niedrigalkyl und Niedrigalkoxy gerade oder verzweigte Gruppen mit 1 bis 4 Kohlenstoffatomen umfassen, welches Verfahren dadurch gekennzeichnet ist, daß (1) ein geschütztes Octahydro-1H-isoindol-1-carboxylat mit der Formel

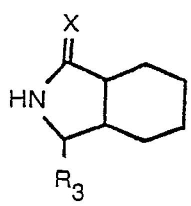

worin $R_3$ eine geschützte Carbonsäuregruppe bedeutet, mit einer N-substituierten Aminosäure der Formel

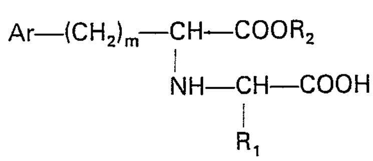

worin $R_1$, $R_2$, Ar und m der zuvor angegebenen Definition entsprechen, einer Peptidkupplung unterzogen wird und (2) die Schutzgruppe entfernt wird.

2. Verfahren zur Herstellung eines substituierten Acylderivats von Octahydro-1H-isoindol-1-carbonsäure oder -ester mit der Formel

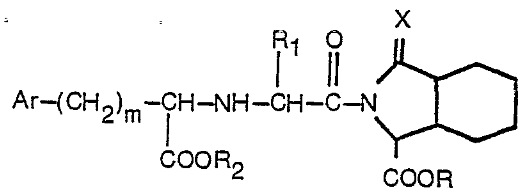

worin Ar, R, $R_1$, $R_2$, m und X der in Anspruch 1 angegebenen Definition entsprechen, welches Verfahren dadurch gekennzeichnet ist, daß eine Verbindung mit der Formel

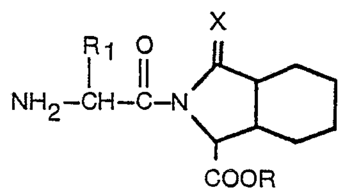

worin R, $R_1$ und X der in Anspruch 1 angegebenen Definition entsprechen, mit einer Verbindung der Formel

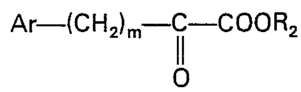

oder der Formel

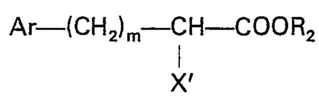

worin Ar, $R_2$ und m der in Anspruch 1 angegebenen Definition entsprechen und X' für Halogen steht, umgesetzt wird.

3. Verfahren zur Herstellung eines pharmazeutisch akzeptablen Salzes einer nach einem Verfahren nach Anspruch 1 oder 2 hergestellten Verbindung, welches Verfahren dadurch gekennzeichnet ist, daß die Verbindung mit einer Base oder einer Säure, die ein pharmazeutisch akzeptables Salz bilden kann, umgesetzt wird.

4. Verfahren nach irgendeinem der Ansprüche 1 bis 3, welches ein Verfahren zur Herstellung eines Derivats der Formel

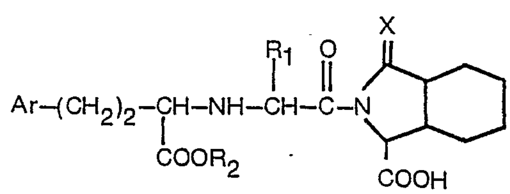

ist, worin $R_1$ Wasserstoff oder Niedrigalkyl mit 1 bis 3 Kohlenstoffatomen bedeutet, $R_2$ Wasserstoff oder Niedrigalkyl mit 1 bis 3 Kohlenstoffatomen bedeutet, Ar Phenyl oder in der para-Position durch Fluor, Chlor, Brom, Methyl, Hydroxy, Methoxy oder Amino substituiertes Phenyl bedeutet und X der in Anspruch 1 angegebenen Definition entspricht, oder eines pharmazeutisch akzeptablen Salzes desselben.

5. Verfahren nach irgendeinem der Ansprüche 1 bis 3, welches ein Verfahren zur Herstellung eines Derivats mit der Formel

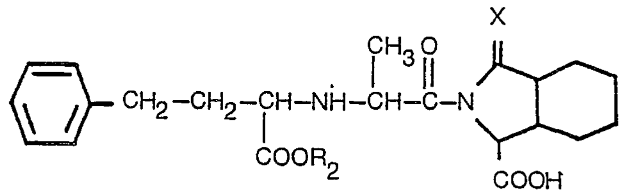

ist, worin $R_2$ Wasserstoff oder Niedrigalkyl mit 1 bis 3 Kohlenstoffatomen bedeutet und X der in Anspruch 1 angegebenen Bedeutung entspricht, oder eines pharmazeutisch akzeptablen Salzes desselben.

6. Verfahren nach irgendeinem der Ansprüche 1 bis 3, welches ein Verfahren zur Herstellung der Verbindung 2-[2-[1Carboxy-3-phenylpropyl)-amino]-1-oxopropyl]-octahydro-3-oxo-1H-isoindol-1-carbonsäure oder eines pharmazeutisch akzeptablen Salzes derselben ist.

7. Verfahren nach irgendeinem der Ansprüche 1 bis 3, welches ein Verfahren zur Herstellung der Verbindung 2-[2[(1-Carbethoxy-3-phenylpropyl)-amino]-1-oxopropyl]-octahydro-3-oxo-1H-isoindol-1-carbonsäure oder eines Pharmazeutisch akzeptablen Salzes derselben ist.